(19) Europäisches Patentamt
European Patent Office
Office européen des brevets

(11) Publication number: **0 129 932**
**A1**

(12) **EUROPEAN PATENT APPLICATION**

(21) Application number: 84200877.3

(22) Date of filing: 20.06.84

(51) Int. Cl.[4]: C 07 C 103/76
C 07 C 102/00, A 61 K 49/04

(30) Priority: 22.06.83 GB 8316965

(43) Date of publication of application:
02.01.85 Bulletin 85/1

(84) Designated Contracting States:
AT BE CH DE FR GB IT LI LU NL SE

(71) Applicant: DAGRA N.V.
Verrijn Stuartweg 60
NL-1112 AX Diemen(NL)

(72) Inventor: Korver, Johannes Anthonius
Albertine Agneslaan 7
NL-1411 ES Naarden(NL)

(74) Representative: Kooy, Leendert Willem et al,
OCTROOIBUREAU VRIESENDORP & GAADE P.O. Box 266
NL-2501 AW The Hague(NL)

(54) **N,N,N',N'-tetra (hydroxyethyl)-5-acetylamino-2,4,6-triiodoisophtaloyldiglycinamide, a process for the preparation of said compound and an X-ray contrast agent containing said compound.**

(57) The water soluble, non-ionic compound N,N,N',N'-tetra (hydroxyethyl)-5-acetylamino -2,4,6- triiodoisophtaloyldiglycinamide is an X-ray contrast agent which is simple to prepare and easy to use.

EP 0 129 932 A1

N,N,N',N'-tetra(hydroxyethyl)-5-acetylamino-2,4,6-triiodoisophtaloyl-diglycinamide, a process for the preparation of said compound and an X-ray contrast agent containing said compound.

The invention relates to the new water soluble nonionic compound N,N,N',N'-tetra(hydroxyethyl)-5-acetylamino-2,4,6-triiodoisophtaloyl-diglycinamine, to a process for the preparation of said compound and to an X-ray contrast agent containing said compound. Water soluble, nonionic compounds constitute a new development in the field of radiodiagnostics.

Aqueous solutions of the non-toxic salts of triiodobenzoic acid derivatives, used for e.g. angiography and urography, are well-known. Generally these solutions are tolerated and have a low toxicity. A disadvantage however is the high osmolality in the required diagnostic concentration, which is a multiple of the osmolality of blood and which may cause undesired side-effects. Half of the osmolality originates from ions that are indispensable for the solubility, but that do not contribute to the contrast activity.

So the search for new compounds was started, of which aqueous solutions could be prepared of the required iodine content but in lower molar or ionic concentrations.

Thus, compounds of the ionic type were synthesized, consisting of two triiodobenzene moieties and one acidic function. On salification of such a hexaiodo mono acid with meglumine or sodium, an average of three

iodine atoms per dissolved particle is obtained, and the molar concentration is only half of same of the comparable triidobenzoates. Examples of this type are described in the Swiss patent specification No. 607 995.

Also triiodinated bases were prepared and combined with the conventional triiodobenzoic acids as described in the European patent specification No. 0022744. In the salts thus obtained, both cation and anion contribute to the contrast activity and the ballast of sodium and/or meglumine is avoided.

Water soluble nonionic compounds have been prepared as described in the British patent specifications 1321591 and 1472050 and the U.S. patent specification 4021481. The presence of a number of hydroxyl groups plays a decisive role in the hydrophylic character and water solubility.

Examples are:

N-(N-methyl-3,5-diacetylamino-2,4,6-triiodobenzoyl)-glucamine, [metrizamide];

N,N'-bis (1,3-dihydroxypropyl)-5-lactylamino-2,4,6-triiodoisophtalamide [iopamidol];

N,N'-bis(2,3-dihydroxypropyl)-5-[N-(2,3-dihydroxypropyl)acetyl-amino]-2,4,6-triiodoisophtalamide [iohexol].

Water soluble and nonionic dimer compounds are also described, consisting of two triiodobenzene moieties, thus containing six iodine atoms per molecule. Their application is however limited because of the high viscosity of their solutions.

The use of iodine compounds with low osmolality is an important improvement in radiography. A disadvantage however is the rather high cost of these compounds, if compared with the conventional contrast agents. This can easily be understood, because the manufacturing procedure comprises more steps and the raw materials are generally more expensive. Apart from this, the isolation and final purification require more effort in connection with the high solubility in water.

The present invention relates to a new water soluble nonionic compound, that can be prepared in the required purity in a simple way at relatively low cost.

This compound is:

N,N,N',N'-tetra(hydroxyethyl)-5-(acetylamino-2,4,6,-triiodoisophtaloyl-bis(glycinamide)

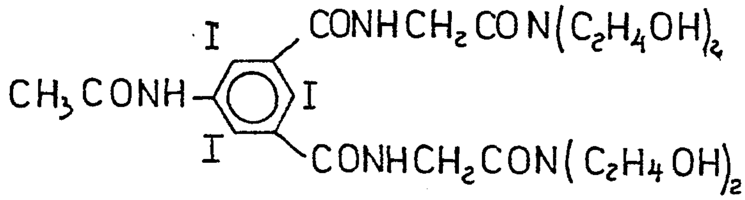

Similar derivatives are already described in the US patent specification No. 4307072 with the general formula

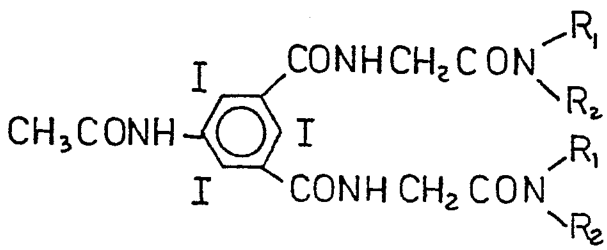

in which $R_1$ is hydrogen, lower, alkyl, hydroxy-lower alkyl or polyhydroxy lower alkyl and $R_2$ is polyhydroxy lower alkyl.

In the final manufacturing step for the preparation of these compounds the dimethyl ester of the corresponding acid is converted with an amine of the type $R_1$-NH-$R_2$ into the diamide.

The simplest amines that can be used are 1-amino-2,3-propanediol and 1-methylamine-2,3-propanediol.

Both the price of these amines and the rather low yield of purification which is reported, to be less than 60%, will increase the cost of manufacturing considerably.

If however the dimethyl ester is allowed to react with the cheap raw material diethanolamine, the bis(diethanolamide is obtained in a high yield and a practically pure form, as described in the examples. The

purification is easy and consist merely of washing the product with methanol in order to remove any adhering diethanol amine.

The invention therefore also relates to a process for the preparation of N,N,N'N'-tetra(hydroxyethyl)-5-acetylamino-2,4,6-triiodoisophtaloxylglycinamide which is characterized in that 5-nitro-isophtaloxylglycine is reduced, the formed amino compound is iodinated, the formed triiodo compound is esterified, the formed ester is acetylated and the acetylated ester is converted with diethanolamino.

The compound is sparingly soluble in methanol but extremely well in water. Its solubility in water at room temperature is about 3 g/ml, much higher than needed for practical purposes.

The intravenous toxicity was determined in mice, after administration of a solution of 300 mg I/ml at an injection rate of 20 g I/kg/min. The LD50 value was found to be 15.4 g I/kg.

Both the method of preparation and yields of 5-acetylamino-2,4,6-triiodoisophtaloyldiglycine dimethyl ester, as described in the US patent specification 3102880 and 4307072, are considerably improved if esterification of the 5-amino-2,4,6-triiodoisophtaloyldiglucine is carried out first and acetylation of the dimethyl ester is carried out subsequently.
Surprisingly it also appears that no special purification of the intermediates is necessary, which also contributes to higher yields. The diiodo by-products, present in the crude iodination product, as a result of some steric hindrance, are for the greater part removed by esterification procedure.
The remaining diiodo product and some inevitable mono-ester is removed by the acetylation procedure.
The remaining mono-ester will be converted into mono-amide, which is completely removed in the amidification step.

By the procedure, as further explained in the examples, an excellent, water soluble, nonionic contrast agents is prepared at low cost, using simple and cheap raw materials in a rapid and simple way.

EXAMPLE 1

1. Preparation of 5-amino-2,4,6-triiodoisophtaloyldiglycine

73.12 g (225 mole) of 5-nitro-isophtaloyldiglycine is dissolved in 1 l of water with ammonia to a pH = 7.5.
20 g of Raney nickel is added and the reduction carried out under hydrogen pressure at 20 atm.
The dolution is filtered and diluted to 3 l.
150 ml 36% HCl is added and the solution is heated to 70°C.
525 ml 1.62 M $NaICl_2$ solution is added. A heavy precipitate is gradually formed. After stirring for 20 hours at 70°C the crystalline product is filtered and washed with bisulphite solution and water.
Yield: 131.5 g. Mol.wt found: 640, theory: 673.

2. Preparation of 5-amino-2,4,6-triiodoisophtaloyldiglycine dimethyl ester

202 g of the crude acid is refluxed for 6 hours in 1 l of methanol, containing 27 g HCl (0.75 N). Both acid and ester are insoluble in methanol. The appearance of the suspension gradually changes. After 6 hours the suspension is filtered and washed with methanol. 198.5 g of an off-white product is obtained. Yield: 94%. Melting point: 254°C. A pure reference sample melts at 266°C. I%: 52.8%, theory: 54,35%. The product still contains about 10% of diiodo compound, together with 3.5% of mono-ester.

3. Preparation of 5-acetylamino-2,4,6-triiodoisophtaloyldiglycine dimethyl ester.

150 g of the crude amino ester is mixed with 300 ml of acetic acid and 75 ml of acetic anhydride. The suspension is stirred and heated to 95°C, 5 ml of sulphuric acid is added, upon which the temperature rises at once to 105°C. the mixture is stirred for another hour at 115°C and cooled. A rather thick suspension is obtained. This is filtered and washed with acetic acid until as much liquid has been collected as was used in the reaction. The crystalline mass is stirred in 400 ml of methanol and filtered. Finally the product is washed with methanol.

129.2 g of a perfectly white product is obtained. Yield: 81% I% : 51.3%, theory: 51.3%. The product contains 2.5% of mono-ester.

4. Preparation of N,N,N',N'-tetra(hydroxyethyl)-5-acetylamino-2,4,6-triiodoisophtaloyldiglycineamide

100 g (134.6 mole) of the di-ester and 100 g of 94% diethanolamine (896 mmol) are stirred at 85°C.
Initially the mixture is a thick suspension, which gradually becomes less viscous. After a short, transient dissolution, a viscous paste is formed. This paste is diluted with 100 ml of methanol and stirred until completion of the reaction. In order to control if the amidification reaction is finised, a small sample of the mixture is mixed with a few drops of water. This should give a clear solution. It takes about 8 hours to complete the reaction.

The mixture is diluted with 400 ml of methanol in order to facilitate the filtration and the removal of the excess of diethanolamine. The filtered product is suspended in 400 ml of methanol at 60°C and stirred for an hour, filtered and washed with methanol and the procedure repeated once more.
Yield: 107.7 g, 90.0%
I%: 42.8%, theory: 42.85%
alkalimetry: absence of mono-amide TLC: one spot
NMR spectrum: in compliance with the predicted structure.

EXAMPLE 2

Preparation of a solution, containing 200 mg I/ml

Into a vial containing 7.00 g of freeze-dried tetra(hydroxyethyl)-5-acetylamino-2,4,6-triiodoisophtaloyldiglycinamide, 11.5 ml of a sterile solvent is injected, consisting of a solution of

| | | |
|---|---|---|
| tris(hydroxymethyl)aminomethane | | 2.25 mg |
| edetate calcium disodium | | 1.5 mg |
| hydrochloric acid to Ph 7.1 | | |
| in water for injection | to | 15 ml |

The vial is gently shaked, until its contents are dissolved.

Thus 15 ml of a solution containing 200 mg I/ml is obtained.

CLAIMS

1. N,N,N',N'-tetra(hydroxyethyl)-5-acetylamino-2,4,6-triiodoisophtaloyldiglycinamide.

2. A process for the preparation of N,N,N',N'-tetra(hydroxyethyl)-5-acetylamino-2,4,6-triiodoisophtaloyldiglycinamide, characterized in that 5-nitro-isoptaloyldiglycine is reduced, the formed amino compound is iodinated, the formed triiodocompound is esterified, the formed ester is acetylated and the acetylated ester is converted with diethanolamine.

3. X-ray contrast agent containing a triiodo compound characterized in that the triiodo compound consists of N,N,N',N'-tetra(hydroxyethyl)-5-acetylamino-2,4,6-triiodoisoptaloyldiglycinamide.

Kooy/DiS

European Patent Office

**EUROPEAN SEARCH REPORT**

0129932
Application number

EP 84200877.3

## DOCUMENTS CONSIDERED TO BE RELEVANT

| Category | Citation of document with indication, where appropriate, of relevant passages | Relevant to claim | CLASSIFICATION OF THE APPLICATION (Int Cl 3) |
|---|---|---|---|
| D,A | US - A - 4 307 072 (K.R. SMITH)<br>* Examples *<br>-- | 1-3 | C 07 C 103/76<br>C 07 C 102/00<br>A 61 K 49/04 |
| D,A | US - A - 3 102 880 (R.D. RANDS, JR.)<br>* Columns 1,2 *<br>---- | 1-3 | |

TECHNICAL FIELDS SEARCHED (Int Cl 3)

C 07 C 103/00

The present search report has been drawn up for all claims

| Place of search | Date of completion of the search | Examiner |
|---|---|---|
| VIENNA | 14-09-1984 | HOFBAUER |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503. 03 82